# EUROPEAN PATENT APPLICATION

(11) **EP 3 336 111 A1**
(43) Date of publication of application: **20.06.2018**
(21) Application number: 16204669.2
(22) Date of filing: 16.12.2016
(51) Int. Cl.: C08F 4/20, C08F 112/08, C08F 112/12, C08F 116/18

(54) **INITIATOR SYSTEM FOR CATIONIC POLYMERIZATION OF OLEFINS**

(71) Applicant: ARLANXEO Canada Inc., Sarnia, Ontario ON N7NT (CA); The University of British Columbia, Vancouver, British Columbia V6T 1Z3 (CA)
(72) Inventor: Arsenault, Gilles, London, Ontario N6G 5K3 (CA); Hazin, Khatera, Vancouver, BC V6T 1Z1 (CA); Gates, Derek, Vancouver, BC V6T 1Z1 (CA)
(74) Representative: Deblon, Jörg-Stephan

(57) **Abstract**

A Brønsted-Lowry acid initiator system for cationic polymerization of an ethylenically unsaturated monomer involves an initiator having a structure of Formula (I) in an anhydrous polymerization medium: 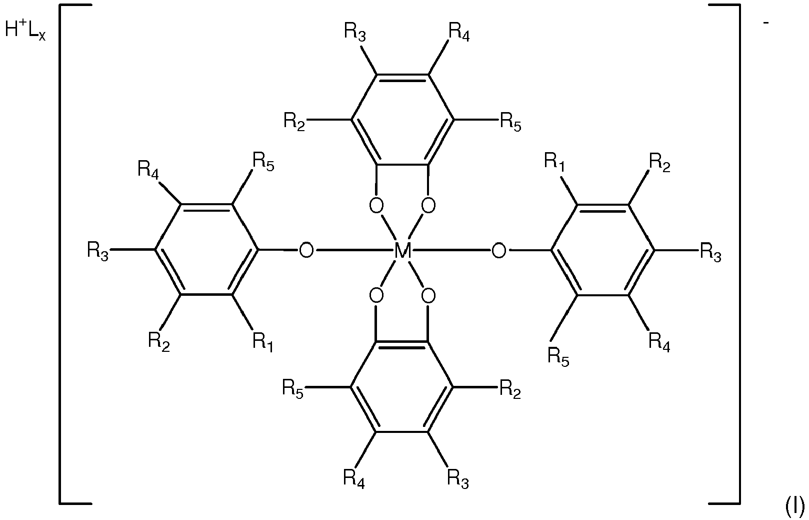 where: M is tantalum (Ta), vanadium (V) or niobium (Ni); each R₁ is independently H, OR₆, F, Cl, Br, I or alkyl, where R₆ is H or alkyl; R₂, R₃, R₄ and R₅ are the same or different and are independently H, F, Cl, Br, I, alkyl or aryl, or two or more of R₂, R₃, R₄ and R₅ on a same benzene ring are taken together to form a bicyclic, tricyclic or tetracyclic moiety with the benzene ring, with the proviso that all of R₂, R₃, R₄ and R₅ on the same benzene ring are not H; L is absent or a molecule that coordinates to H⁺; and, x is 0 when L is absent, or x is 0.5 or more when L is present.

## Description

### Field

This application relates to a process for producing a polymer from one or more ethylenically unsaturated monomers. The application further relates to an initiator system for the process, and to compounds in the initiator system.

### Background

Various types of initiator systems for cationic polymerization of ethylenically unsaturated monomers are known in the art, including systems based on protonic or Brønsted-Lowry acids, Lewis acids (e.g. Friedel-Crafts catalysts), carbenium ion salts and ionizing radiation. Common protonic acids include phosphoric, sulfuric, fluoro-, and triflic acids, which tend to produce only low molecular weight polymers.

Lewis acids are the most common compounds used for initiation of cationic polymerization, and include, for example, SnCl₄, AlCl₃, BF₃ and TiCl₄. Although Lewis acids alone may be able to induce polymerization, the reaction occurs much faster with a co-initiator that acts as a suitable cation source (e.g. water, alcohols, HCl). However, such cationic polymerization reactions generally require very low temperature (about -100°C to about -90°C) to produce polymers of suitable molecular weight. Further, polymerization processes performed at such low temperatures are energy intensive; therefore, a process that can produce polymers with similar molecular weights at higher temperatures would significantly reduce the energy consumption and manufacturing cost of the process.

Recently, an initiator system for cationic polymerization has been developed based on a pentavalent phosphorus (V) complex with a dihydroxy compound (United States Patent Publication US 2012/0208971 published August 16, 2012). However, this initiator system produces low molecular weight products at higher temperatures, requiring lower temperatures to produce polymers of desirably high molecular weight. For example, the polymerization of *α*-methyl styrene at -50°C produces poly(α-methylstyrene) having Mₙ of less than about 7000 g/mol, Further, in order to produce polystyrene having Mₙ of greater than 100,000 g/mol, the polymerization must be done at temperatures lower than -80°C. The phosphorus complex can also be difficult to handle due to lack of stability.

There remains a need for initiator systems for cationic polymerization, which can produce suitably high molecular weight polymer at higher temperatures.

### Summary

A strong Brønsted-Lowry acid based on complexes of tantalum (V) ions or other isoelectronic metal ions (e.g. vanadium (V) or niobium (V) ions) provides an efficient initiator system for cationic polymerization of ethylenically unsaturated monomers at higher temperatures than existing initiator systems for cationic polymerization. High molecular weight polymers may be formed with the use of the present initiator system at higher temperatures.

In one aspect, there is provided a process for producing a polymer, the process comprising polymerizing one or more ethylenically unsaturated monomers under anhydrous conditions in presence of a Brønsted-Lowry acid polymerization initiator, the Brønsted-Lowry acid polymerization initiator having a structure of Formula (I): 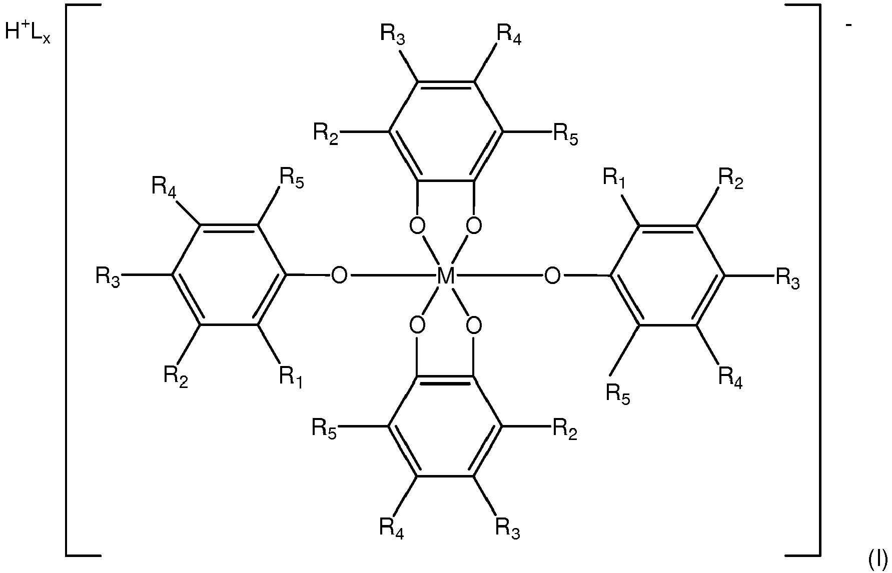 where:
M is tantalum (Ta), vanadium (V) or niobium (Ni);
each R₁ is independently H, OR₆, F, Cl, Br, I or alkyl, where R₆ is H or alkyl;
R₂, R₃, R₄ and R₅ are the same or different and are independently H, F, Cl, Br, I, alkyl or aryl, or two or more of R₂, R₃, R₄ and R₅ on a same benzene ring are taken together to form a bicyclic, tricyclic or tetracyclic moiety with the benzene ring, with the proviso that all of R₂, R₃, R₄ and R₅ on the same benzene ring are not H;
L is absent or a molecule that coordinates to H⁺; and,
x is 0 when L is absent, or x is 0.5 or more when L is present.

In another aspect, there is provided a Brønsted-Lowry acid initiator system for cationic polymerization of an ethylenically unsaturated monomer, the Brønsted-Lowry acid initiator system comprising an initiator having a structure of Formula (I) as defined above in an anhydrous polymerization medium.

In another aspect, there is provided a compound of Formula (I), where M, R₁, R₂, R₃, R₄, R₅, L and x are as defined above.

Further features will be described or will become apparent in the course of the following detailed description. It should be understood that each feature described herein may be utilized in any combination with any one or more of the other described features, and that each feature does not necessarily rely on the presence of another feature except where evident to one of skill in the art.

### Detailed Description

The strong Brønsted-Lowry acid comprises a metal complex of organic ligands as described above for Formula (I). Alkyl is preferably C₁₋₆ alkyl, more preferably C₁₋₄ alkyl (e.g. methyl, ethyl, n-propyl, *i*-propyl, n-butyl, s-butyl, *t*-butyl), even more preferably methyl. Alkyl may be unsubstituted or substituted by one or more substituents. Substituents may be, for example, F, Cl, Br or aryl. Aryl is preferably C₁₋₁₈ aryl, more preferably C₁₋₁₀ aryl, even more preferably C₁₋₆ aryl, for example phenyl. Aryl may be unsubstituted or substituted by one or more substituents. Substituents may be, for example, F, Cl, Br or alkyl, where alkyl is as defined above.

M is preferably tantalum.

R₁ is preferably independently H, OH or CH₃. More preferably, each R₁ is the same and is H, OH or CH₃.

When two or more of R₂, R₃, R₄ and R₅ on a same benzene ring are taken together to form a bicyclic, tricyclic or tetracyclic moiety with the benzene ring, the moiety is preferably a fused ring system, for example a naphthyl moiety or an anthracyl moiety. R₂, R₃, R₄ and R₅ are preferably independently H, F, Cl, Br, I, alkyl or aryl, with the proviso that all of R₂, R₃, R₄ and R₅ on the same benzene ring are not H. More preferably, R₂, R₃, R₄ and R₅ are independently H, F, Cl or Br, with the proviso that all of R₂, R₃, R₄ and R₅ on the same benzene ring are not H. When R₂, R₃, R₄ and R₅ on the same benzene ring are both hydrogen and halogen (e.g. F, Cl, Br), the benzene ring may be mono-, di- or tri-halogenated. More preferably, R₂, R₃, R₄ and R₅ are independently F or Cl. Even more preferably, R₂, R₃, R₄ and R₅ are the same and are F or Cl.

In one embodiment, M is Ta; each R₁ is H, F, Cl, OH or CH₃; R₂, R₃, R₄ and R₅ are F or Cl; L is Et₂O and x is 2.

In another embodiment, M is Ta; each R₁ is H, OH, OCH₃ or CH₃; R₂, R₃, R₄ and R₅ are F or Cl; L is Et₂O and x is 2.

The Brønsted-Lowry acid polymerization initiator is particularly useful for initiating the polymerization or copolymerization of ethylenically unsaturated monomers. Ethylenically unsaturated monomers are compounds having at least one olefin bond therein. The monomers preferably comprise from 2 to 20 carbon atoms. Some examples of ethylenically unsaturated monomers include alkyl vinyl compounds (e.g. alkyl vinyl ethers and the like) and aryl vinyl compounds (e.g. styrene, *α*-methylstyrene, *p-*methylstyrene, *p*-methoxystyrene, 1-vinylnaphthalene, 2-vinylnaphthalene, 4-vinyltoluene and the like). Of particular note are n-butyl vinyl ether, styrene and *α*-methylstyrene.

Polymers formed from the polymerization of the monomers may be homopolymers, copolymers, terpolymers or other forms of polymers. The polymers may be linear or branched (e.g. star branched). Mixtures of two or more monomers may be polymerized into copolymers or terpolymers. Some examples of polymers include polystyrene, poly(*α*-methylstyrene), poly(N-vinylcarbazole), polyterpenes and the like. Of particular note are polystyrenes (e.g. polystyrene and poly(α-methylstyrene) and poly(n-butyl vinyl ether).

Polymers produced in the polymerization of ethylenically unsaturated monomers may have number average molecular weights (Mₙ) of at least about 2,000 g/mol, or at least about 5,000 g/mol, or at least about 10,000 g/mol, or at least about 20,000 g/mol, or at least about 30,000 g/mol, or at least about 50,000 g/mol, or at least about 100,000 g/mol, depending on the monomer or momomers undergoing polymerization, the relative amounts of monomer and initiator, the temperature at which the polymerization is conducted and other process conditions. The polymer may have number average molecular weights (Mₙ) up to about 1,000,000 g/mol, or up to about 500,000 g/mol, or up to about 250,000 g/mol.

The initiator is a cationic initiator because the initiator is a Brønsted-Lowry acid, thereby further comprising a hydrogen ion (H⁺) as counterion to an anionic metal complex. The hydrogen ion may be associated as a "naked" ion with the metal complex (i.e. x = 0). To stabilize the hydrogen ion, the initiator may further comprise a stabilizing molecule (L) for the hydrogen ion. The value of x may be an integer or a fractional number depending on whether H⁺ ions associated with neighboring complexes in a bulk material of the polymerization initiator share a molecule, L. When a molecule L is shared between neighboring H⁺ ions, the value of x may be fractional. The value of x is preferably 0.5, 1, 1.5, 2, 2.5 or 3. In one embodiment, there are two stabilizing molecules for each H⁺ ion (i.e. x = 2). The stabilizing molecule may be a molecule that can form hydrogen bonds with the hydrogen ion. The stabilizing molecule may therefore contain one or more atoms that have lone pairs of electrons, for example O or N atoms. Some examples of stabilizing molecules include ethers, alcohols, amines and the like. Aprotic stabilizing molecules are preferred. Alkyl and cycloalkyl ethers are particularly preferred, for example tetrahydrofuran, tetrahydropyran, dioxane, dimethyl ether, diethyl ether, dipropyl ether, diisopropyl ether, methyl ethyl ether, methyl n-propyl ether, methyl isopropyl ether, methyl tert-butyl ether, ethyl tert-butyl ether, dihexyl ether, 2,5-dimethyltetrahydrofuran, 2-chloro ethyl ether, 2-methyltetrahydrofuran, cyclopentyl methyl ether, diethylene glycol dimethyl ether (diglyme), tetraethylene glycol dimethyl ether and the like. In one embodiment, the stabilizing molecule is diethyl ether. Where the stabilizing molecule is a solvent, the stabilizing molecule may form a solvate with the hydrogen ion.

The compound of Formula (I) may be synthesized by contacting a metal ion precursor compound in a reaction mixture with an organic α-,β-dihydroxy ligand compound of Formula (IIa), or by contacting the metal ion precursor compound with an organic α-,β-dihydroxy ligand compound of Formula (IIa) and an organic monohydroxy ligand compound of Formula (IIb): 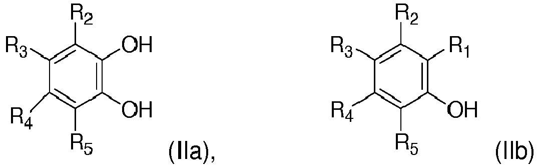 where R₁ and R₂ are as defined above, with the proviso that R₁ in the compound of Formula (IIb) is not OH. Mixtures of different organic ligand compounds may be used. The metal ion precursor compound and organic ligand compounds may be present in the reaction mixture in amounts to provide a molar ratio that results in the metal complex having sufficient ligands to provide a negative charge to the metal complex. To provide metal complexes where R₁ is OH, about 4 molar equivalents of the organic α-,β-dihydroxy ligand compound of Formula (IIa) is suitable to result in the metal complex having four ligands, two bidentate ligands and two monodentate ligands, thereby providing the metal complex with a -1 charge. To provide metal complexes where R₁ is other than OH, about 2 molar equivalents of the organic α-,β-dihydroxy ligand compound of Formula (IIa) and 2 molar equivalents of the organic monohydroxy ligand compound of Formula (IIb) are suitable to result in the metal complex having four ligands, two bidentate ligands and two monodentate ligands, thereby providing the metal complex with a -1 charge. Where both the compounds of Formula (IIa) and (IIb) are reacted with the metal ion precursor compound, the compound of Formula (IIa) is preferably reacted first with the metal ion precursor compound to produce an intermediate metal complex, followed by further reaction with the compound of Formula (IIb) to produce the compound of Formula (I).

The metal ion precursor compound may be a compound of a metal ion with leaving groups as ligands. Suitable leaving groups include, for example, halogen (Cl, Br), CO, CN and the like. The metal ion precursor compound and organic ligand compounds are preferably dry and high purity. Contacting the metal ion precursor compound with the organic ligand compounds may be performed in the presence or absence of a solvent, preferably in the presence of a solvent. The solvent may comprise an aprotic organic solvent, preferably a non-coordinating solvent. Some examples of suitable solvents include alkyl halides (e.g. dichloromethane), aromatic hydrocarbons (e.g. toluene) and acetonitrile. A stabilizing molecule for hydrogen ions may be included in the reaction mixture, preferably after the metal complex is formed, to solvate the hydrogen ion. The reaction is preferably conducted under anhydrous conditions. The reaction may be conducted at elevated temperature, for example by refluxing the solvent. The reaction may be conducted for an amount of time sufficient to maximize the yield of the initiator, for example for a time up to about 3 hours. The reaction is preferably conducted by slowly adding the ligand compound to a reaction mixture containing the metal ion precursor compound, although other addition schemes may be used. The initiator may be recovered from the reaction mixture by standard techniques, for example filtration, washing, recrystallization and drying.

The initiator is preferably used in amount to provide a monomer to initiator mole ratio ([M]:[I]) of at least about 20:1. A higher [M]:[I] may be preferred in some embodiments to produce high yields of high molecular weight polymer. In some embodiments, the [M]:[I] may be at least about 100:1. In some embodiments, the [M]:[I] may be in a range of about 100:1 to about 1000:1, or about 200:1 to about 800:1, or about 300:1 to about 500:1.

The polymerization is generally conducted in a polymerization medium. The polymerization medium may be provided, for example, by a solvent or diluent. Solvents or diluents for the polymerization may include, for example a halogenated organic liquid, a non-halogenated organic liquid or mixtures thereof. Halogenated organic liquids include, for example, chlorinated or fluorinated organic compounds. Chlorinated organic compounds include, for example C1-C4 alkyl chlorides (e.g. dichloromethane (DCM) and methyl chloride (MeCl)). DCM is generally useful as a solvent for solution polymerization, while MeCl is generally useful as a diluent for slurry polymerization. Fluorinated organic compounds include, for example, hydrofluorocarbons (HFC) such as 1,1,1,2-tetrafluoroethane and the like, and hydrofluorinated olefins (HFO) such as 2,3,3,3-tetrafluoro-1-propene and the like. Fluorinated organic compounds are generally useful as diluents for slurry polymerization. Non-halogenated organic liquids include, for example, aliphatic hydrocarbons (e.g. cyclohexane, cyclopentane, 2,2-dimethylbutane, 2,3-dimethylbutane, 2-methylpentane, 3-methylpentane, n-hexane, methylcyclopentane and 2,2-dimethylpentane). Halogenated organic solvents, in particular C1-C4 alkyl chlorides are preferred. Dichloromethane (CH₂Cl₂) or methyl chloride (MeCl) are particularly preferred.

The solvent or diluent is preferably present in the polymerization medium in an amount of about 10-80 vol%, based on volume of the polymerization medium. In preferred embodiments, the medium may comprise a diluent in an amount of about 55-80 vol%, or a solvent in an amount of about 10-50 vol%.

The polymerization is conducted under anhydrous conditions. Preferably, water is present in an amount less than about 1 ppm, more preferably less than about 0.5 ppm, yet more preferably less than about 0.1 ppm. It is preferable to eliminate water from the polymerization medium altogether. Reducing or eliminating moisture in the polymerization medium helps to produce polymers having higher molecular weights at higher yields.

It is an advantage of the present initiator system that the polymerization may be conducted at a higher temperature than with other Brønsted-Lowry acid or Lewis acid initiator systems, while being able to produce suitably high molecular weight polymers at good yield. The temperature at which the polymerization is conducted may be -90°C or higher, or -85°C or higher, or -80°C or higher, or -70°C or higher, or -60°C or higher, or -50°C, or -40°C or higher. The temperature may be as high as 30°C or lower, or 20°C or lower, or 10°C or lower, or 0°C or lower, or -10°C or lower, or -15°C or lower, or -20°C or lower, or -25°C or lower, -30°C or lower, or -35°C or lower.

### EXAMPLES:

### General materials and procedures:

All experiments were performed using standard Schlenk or glove box techniques under nitrogen atmosphere.

Dichloromethane (CH₂Cl₂) and diethyl ether (Et₂O) were deoxygenated with nitrogen and dried by passing through a column containing activated alumina. CH₂Cl₂ (Sigma Aldrich), Et₂O (Fisher Scientific), styrene (Sigma Aldrich) and n-butyl vinyl ether (Sigma Aldrich) were dried over calcium hydride, distilled and freeze-pump-thaw (x3) degased prior to use. CH₂Cl₂ and Et₂O were stored over molecular sieves prior to use.

Tantalum pentachloride (Aldrich) was used without further purification. Tetrachlorocatechol was prepared following the procedure described in Lübbecke H., Boldt P. Tetrahedron 1978, 34, 1577-1579, the contents of which is herein incorporated by reference, and then azeotropically distilled and recrystallized from hot toluene prior to use.

¹H and ¹³C{¹H} NMR spectra were recorded on Bruker Avance 300 or 400 MHz spectrometers at room temperature unless noted. ¹H NMR and ¹³C{¹H} NMR spectra were referenced to deuterated solvents.

Molecular weight of polymers was determined by triple detection gel permeation chromatography (GPC-LLS) utilizing an Agilent 1260 Series standard auto sampler, an Agilent 1260 series isocratic pump, Phenomenex Phenogel™ 5 µm narrowbore columns (4.6 x 300 mm) 10⁴ A (5000-500,000), 500 A (1,000-15,000), and 10³ A (1,000-75,000), a Wyatt Optilab™ rEx differential refractometer (λ = 658 nm, 25 °C), as well as a Wyatt tristar miniDAWN (laser light scattering detector (λ = 690 nm)) and a Wyatt ViscoStar viscometer. Samples were dissolved in THF (ca. 2 mg mL⁻¹) and a flow rate of 0.5 mL min⁻¹ was applied. The differential refractive index (dn/dc) of poly(n-butyl vinyl ether) (dn/dc = 0.068 mL g⁻¹) in THF was calculated by using Wyatt ASTRA software 6.1. The differential refractive index (dn/dc) of poly(styrene) (dn/dc = 0.185 mL g⁻¹) and of poly(α-methylstyrene) (dn/dc = 0.204 mL g⁻¹) has been reported in McManus NT, Penlidis A. J. Appl. Polym. Sci. 1998, 70, 1253-1254.

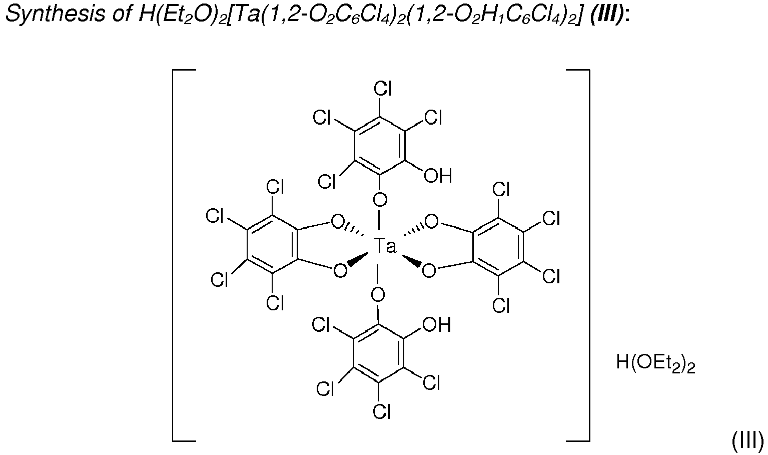

TaCl₅ (0.53 g, 1.5 mmol) was stirred in anhydrous CH₂Cl₂ (10 mL) and the white suspension was slowly heated to reflux. In another Schlenk flask, about 4 equivalents of tetrachlorocatechol (1.48 g, 5.9 mmol) was prepared in warm anhydrous CH₂Cl₂ (14 mL) and the bright orange-red solution was added via cannula to the refluxing TaCl₅ solution at 90°C to afford a dark green reaction mixture. After 10 minutes a colorless precipitate was obtained. The reaction mixture was refluxed for 80 minutes and cooled to ambient temperature. Upon addition of Et₂O (25 mL), a green clear solution formed. The solution was cooled in an ice bath to afford an off-white precipitate within 15 minutes. The solid was collected by filtration, washed with CH₂Cl₂ (5 mL) and dried *in vacuo.* Yield = 1.13 g, 0.9 mmol, 66 %. A concentrated solution of the crude product in CH₂Cl₂ afforded colorless crystals of H(Et₂O)₂[Ta(1,2-O₂C₆Cl₄)₂(1,2-O₂H₁C₆Cl₄)₂] **(III)** (-30°C, ca. 3 d).
¹H NMR (400 MHz, CD₂Cl₂, 25°C): δ = 9.37 (br s, 3H, HO & *H*(Et₂O)₂), 4.00 (q, 8H, J = 7 Hz, C*H*₂CH₃), 1.40 ppm (t, 12H, J = 7 Hz, CH₂C*H*₃); ¹H NMR (400 MHz, CD₂Cl₂, -85°C): δ = 16.73 (s, 1 H, *H*(Et₂O)₂), 9.40 (s, 2H, HO), 4.03 (q, 8H, J = 7 Hz, C*H*₂CH₃), 1.38 ppm (t, 6H, J = 7 Hz, CH₂C*H*₃).
¹³C{¹H} NMR (75 MHz, CD₂Cl₂, -85°C): δ = 150.0 (s), 145.3 (s), 144.3 (s), 139.8 (s), 125.0 (s), 121.6 (s), 121.1 (s), 118.6 (s), 116.7 (s), 70.3 (s), 13.3 ppm (s).

Elemental analysis (%) found: C, 28.30; H, 1.80. Calcd. for C₃₂H₂₃Cl₁₆O₁₀TaCH₂Cl₂: C, 28.31; H,1.80.

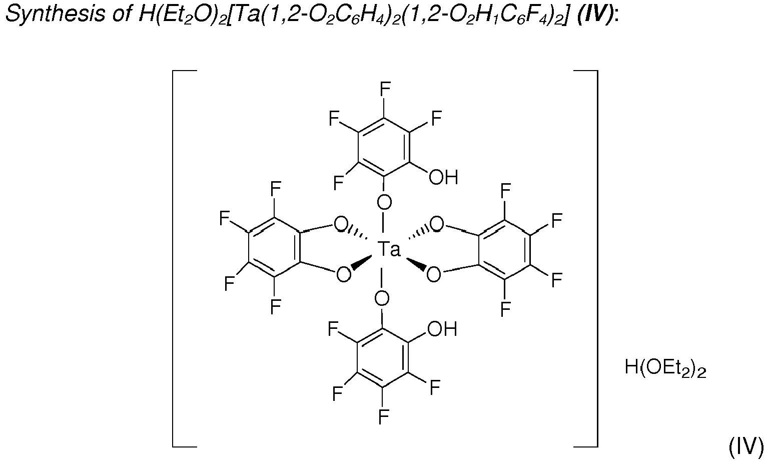

The reaction of 4 equivalents of tetrafluorocatechol with TaCl₅ in a manner as described for the synthesis of the chlorinated analog (III) above may afford the corresponding H(Et₂O)₂[Ta(1,2-O₂C₆H₄)₂(1,2-O₂H₁C₆F₄)₂] (IV).

*Polymerization of n-butyl vinyl ether, styrene and α-methylstyrene using initiator (III):*
Polymerization of n-butyl vinyl ether, styrene and α-methylstyrene monomers with initiator (III) was performed by the following general procedure.

Initiator and monomer are initially stored at -30°C inside a freezer in a glovebox under a positive atmosphere of dry N₂ gas. The initiator (0.010 g, 0.010 mmol) is transferred to a 25 mL Schlenk flask, which is sealed with a rubber septum and then brought outside the glovebox maintaining isolation from the outside atmosphere to be connected to a dry N₂ gas line. The initiator in the flask is cooled to -78°C with an acetone/ dry ice bath. Anhydrous, degassed CH₂Cl₂ (2.0 mL) stored over activated molecular sieves is added via syringe to the initiator under a flow of dry N₂ gas and stirred to guarantee a homogenous solution at -78°C. The mixture is kept at -78°C for 10 minutes, or warmed or cooled to a different desired temperature and held at that temperature for 10 minutes, before addition of the monomer.

Freshly prepared and degassed monomer in an amount to achieve a desired monomer to initiator ratio ([M]:[I]) is collected in a 1 ml single-use plastic syringe inside the glovebox. The monomer is then injected rapidly through the rubber septum on the Schlenk flask into the initiator solution at the desired temperature under a constant flow of dry N₂ gas, and the reaction mixture is continuously stirred for 15 minutes while polymerization occurs. After the 15 minutes, the reaction is quenched with 0.2 mL of a solution of NH₄OH in MeOH (10 vol%), the Schlenk flask is removed from the cooling bath and all volatiles are removed *in vacuo.* The crude product is dissolved in 2 mL CH₂Cl₂ and added one drop at a time via syringe to vigorously stirred MeOH (40 mL) to precipitate an oily residue. The polymer is collected by centrifugation and dried *in vacuo.* Absolute molecular weight (Mₙ) is determined using triple-detection GPC.

### Effect of temperature on n-butyl vinyl ether polymerization

Table 1 shows data for the polymerization of n-butyl vinyl ether using initiator (III) at different temperatures. The data for each example represents the average of at least three separate polymerization reactions. Mₙ calc. = 40,000 g/mol. Table 1 shows that significant yield of poly(n-butyl vinyl ether) having a reasonably high molecular weight (Mₙ) can be achieved at temperatures well above -90°C.

**Table 1**

| Ex. | T (°C) | [M]:[I] | Yield (%) | Mₙ (g/mol) | PDI |
|---|---|---|---|---|---|
| 1 | 18 | 400 | 38 | 19,800 | 1.69 |
| 2 | 0 | 400 | 40 | 17,000 | 1.21 |
| 3 | -15 | 400 | 65 | 25,300 | 1.89 |
| 4 | -38 | 400 | 62 | 30,600 | 1.96 |
| 5 | -50 | 400 | 68 | 29,400 | 2.07 |
| 6 | -78 | 400 | 71 | 32,200 | 1.58 |
| 7 | -84 | 400 | 54 | 39,100 | 1.12 |

### Effect of monomer to initiator ratio ([M]:[I]) on n-butyl vinyl ether polymerization

Table 2 shows data for the polymerization of n-butyl vinyl ether using initiator (III) at different monomer to initiator ratios. The data for each example represents the average of at least three separate polymerization reactions. Table 2 shows that Mₙ of poly(n-butyl vinyl ether) can be increased by increasing [M]:[I] while keeping yield relatively constant.

**Table 2**

| Ex. | T (°C) | [M]:[I] | Yield (%) | Mₙ (g/mol) | PDI |
|---|---|---|---|---|---|
| 8 | -84 | 200 | 36 | 43,800 | 1.09 |
| 7 | -84 | 400 | 54 | 39,100 | 1.12 |
| 9 | -84 | 800 | 52 | 73,000 | 1.13 |

### Effect of temperature on styrene polymerization

Table 3 shows data for the polymerization of styrene using initiator (III) at different temperatures. The data for each example represents the average of at least three separate polymerization reactions. Mₙ calc. = 40,000 g/mol. Table 3 shows that an excellent balance between high yield and high molecular weight of polystyrene can be achieved at temperatures much higher than -90°C.

**Table 3**

| Ex. | T (°C) | [M]:[I] | Yield (%) | Mₙ (g/mol) | PDI |
|---|---|---|---|---|---|
| 10 | 19 | 400 | 67 | 6,100 | 2.16 |
| 11 | 0 | 400 | 82 | 9,500 | 2.94 |
| 12 | -15 | 400 | 85 | 12,800 | 3.51 |
| 13 | -38 | 400 | 72 | 131,500 | 1.34 |
| 14 | -50 | 400 | 78 | 147,100 | 1.43 |
| 15 | -78 | 400 | 7 | 205,600 | 1.29 |

### Effect of the presence of water on styrene polymerization

Table 4 shows data for the polymerization of styrene using initiator (III) at different temperatures in the presence of different amounts of trace moisture. Distilled water in the indicated amounts was added to the anhydrous CH₂Cl₂ prior to polymerization. Table 4 shows that the presence of trace moisture can drastically reduce yield and molecular weight of the polystyrene.

**Table 4**

| Ex. | T (°C) | H₂O (ppm) | [M]:[I] | Yield (%) | Mₙ (g/mol) | PDI |
|---|---|---|---|---|---|---|
| 16 | -50 | 0 | 400 | 78 | 145,000 | 1.43 |
| 17 | -50 | 1 | 400 | 1.6 | 54,800 | 1.42 |
| 18 | -50 | 5 | 400 | 0.9 | 67,700 | 1.58 |
| 19 | -50 | 10 | 400 | 0.8 | 59,900 | 1.62 |
| 20 | -50 | 100 | 400 | 0.6 | 17,110 | 1.84 |
| 21 | -78 | 0 | 400 | 5 | 205,600 | 1.29 |
| 22 | -78 | 1 | 400 | 1.6 | 121,800 | 1.39 |
| 23 | -78 | 5 | 400 | 1.3 | 102,800 | 1.44 |
| 24 | -78 | 10 | 400 | 1.0 | 68,200 | 1.78 |
| 25 | -78 | 100 | 400 | --- | --- | --- |

### Effect of temperature on α-methyl styrene polymerization

Table 5 shows data for the polymerization of *α*-methyl styrene using initiator (III) at different temperatures. The data for each example represents the average of at least three separate polymerization reactions. Mₙ calc. = 40,000 g/mol. Table 5 shows that good balance of high yield and high molecular weight for poly(α-methylstyrene) can be achieved at temperatures much higher than -90°C.

**Table 5**

| Ex. | T (°C) | [M]:[I] | Yield (%) | Mₙ (g/mol) | PDI |
|---|---|---|---|---|---|
| 26 | 19 | 400 | 1 | n.d. | n.d. |
| 27 | 0 | 400 | 3 | 5,100 | 1.63 |
| 28 | -15 | 400 | 45 | 4,800 | 2.37 |
| 29 | -38 | 400 | 63 | 10,100 | 1.87 |
| 30 | -50 | 400 | 82 | 66,400 | 1.81 |
| 31 | -78 | 400 | 56 | 241,000 | 1.32 |

| | | | | | |
|---|---|---|---|---|---|
| n.d. = not determined | | | | | |

The novel features will become apparent to those of skill in the art upon examination of the description. It should be understood, however, that the scope of the claims should not be limited by the embodiments, but should be given the broadest interpretation consistent with the wording of the claims and the specification as a whole.

## Claims

1. A Brønsted-Lowry acid initiator system for cationic polymerization of an ethylenically unsaturated monomer, the Brønsted-Lowry acid initiator system comprising an initiator having a structure of Formula (I) in an anhydrous polymerization medium: 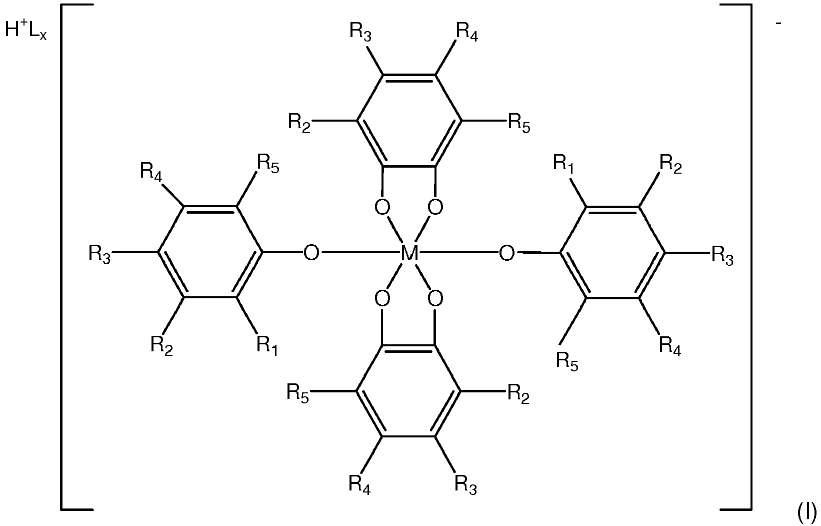 where:
M is tantalum (Ta), vanadium (V) or niobium (Ni);
each R₁ is independently H, OR₆, F, Cl, Br, I or alkyl, where R₆ is H or alkyl;
R₂, R₃, R₄ and R₅ are the same or different and are independently H, F, Cl, Br, I, alkyl or aryl, or two or more of R₂, R₃, R₄ and R₅ on a same benzene ring are taken together to form a bicyclic, tricyclic or tetracyclic moiety with the benzene ring, with the proviso that all of R₂, R₃, R₄ and R₅ on the same benzene ring are not H;
L is absent or a molecule that coordinates to H⁺; and,
x is 0 when L is absent, or x is 0.5 or more when L is present.

2. The system according to claim 1, wherein M is Ta.

3. The system according to claim 1 or 2, wherein L is an alkyl ether or a cycloalkyl ether.

4. The system according to claim 1 or 2, wherein L is diethyl ether.

5. The system according to any one of claims 1 to 4, wherein R₁ is H, F, Cl, OH, OCH₃ or CH₃, and wherein R₂, R₃, R₄ and R₅ are H, F, Cl or Br with the proviso that all of R₂, R₃, R₄ and R₅ on the same benzene ring are not H.

6. The system according to claim 1, wherein: M is Ta; both of R₁ are OH; R₂, R₃, R₄ and R₅ are Cl; L is Et₂O; and, x is 2.

7. The system according to any one of claims 1 to 6, wherein the polymerization medium comprises dichloromethane or methyl chloride.

8. The system according to any one of claims 1 to 7, containing substantially no water.

9. A process for producing a polymer, the process comprising polymerizing one or more ethylenically unsaturated monomers with the initiator system as defined in any one of claims 1 to 8.

10. The process according to claim 9, wherein the polymerization is performed at a temperature of -85°C or higher.

11. A compound of Formula (I): 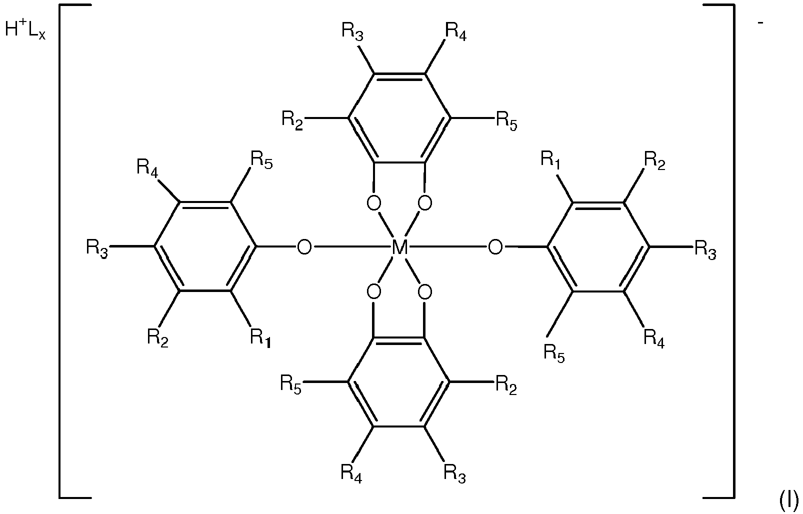 where:
M is tantalum (Ta), vanadium (V) or niobium (Ni);
each R₁ is independently H, OR₆, F, Cl, Br, I or alkyl, where R₆ is H or alkyl;
R₂, R₃, R₄ and R₅ are the same or different and are independently H, F, Cl, Br, I, alkyl or aryl, or two or more of R₂, R₃, R₄ and R₅ on a same benzene ring are taken together to form a bicyclic, tricyclic or tetracyclic moiety with the benzene ring, with the proviso that all of R₂, R₃, R₄ and R₅ on the same benzene ring are not H;
L is absent or a molecule that coordinates to H⁺; and,
x is 0 when L is absent, or x is 0.5 or more when L is present.

12. The compound according to claim 11, wherein M is Ta.

13. The compound according to claim 11 or 12, wherein L is diethyl ether.

14. The compound according to any one of claims 11 to 13, wherein R₁ is H, F, Cl, OH, OCH₃ or CH₃, and wherein R₂, R₃, R₄ and R₅ are H, F, Cl or Br with the proviso that all of R₂, R₃, R₄ and R₅ on the same benzene ring are not H.

15. The compound according to claim 11, wherein: M is Ta; both of R₁ are OH; R₂, R₃, R₄ and R₅ are Cl; L is Et₂O; and, x is 2.
